# EUROPÄISCHE PATENTSCHRIFT

(11) **EP 1 671 541 B1**
(45) Veröffentlichungstag und Bekanntmachung des Hinweises auf die Patenterteilung: **06.04.2016**
(21) Anmeldenummer: 06004673.7
(22) Anmeldetag: 18.09.2001
(51) Int. Cl.: A01N 33/08, A01N 33/12, A01N 33/04

(54) **Desinfektionsmittel**
Disinfectant
Désinfectant

(30) Priorität: 20.09.2000 EP 00120590
(43) Veröffentlichungstag der Anmeldung: 21.06.2006
(62) Teilanmeldung aus: 01985228.4
(73) Patentinhaber: Lonza AG, 4052 Basel (CH)
(72) Erfinder: Lützeler, Michael, 79639 Grenzach-Wyhlen (DE); Ranft, Volker, 79730 Murg (DE)

(56) Entgegenhaltungen:
- EP-A- 0 333 143
- EP-A- 1 025 967
- WO-A-93/15173
- WO-A-98/20732
- WO-A-99/15012
- FR-A- 2 602 955
- DATABASE WPI Section Ch, Week 199946 Derwent Publications Ltd., London, GB; Class A97, AN 1999-541231 XP002190404 & CN 1 222 566 A (CHEN Y) 14. Juli 1999 (1999-07-14)
- DATABASE WPI Section Ch, Week 199824 Derwent Publications Ltd., London, GB; Class D22, AN 1998-266961 XP002190405 & JP 10 087410 A (KANTO KAGAKU KK) 7. April 1998 (1998-04-07)
- DATABASE WPI Section Ch, Week 197749 Derwent Publications Ltd., London, GB; Class D22, AN 1977-87079Y XP002190406 & JP 50 132126 A (ANONYMOUS) 20. Oktober 1975 (1975-10-20)
- DATABASE CA [Online] CHEMICAL ABSTRACTS SERVICE, COLUMBUS, OHIO, US; XP002190402 gefunden im STN-INTERNATIONAL accession no. 105:99132 CA & RO 87 920 A (INTREPRINEREA DE DETERGENTI) 20. Dezember 1985 (1985-12-20)
- DATABASE CA [Online] CHEMICAL ABSTRACTS SERVICE, COLUMBUS, OHIO, US; XP002190403 gefunden im STN-INTERNATIONAL accession no. 134:168324 CA & CN 1 258 448 A (TIANJIN AIHUA FESHENER) 5. Juli 2000 (2000-07-05)
- DATABASE WPI Section Ch, Week 199318 Derwent Publications Ltd., London, GB; Class A97, AN 1993-149118 XP002190407 & JP 05 085905 A (XYENCE KK) 6. April 1993 (1993-04-06)

## Beschreibung

Die Erfindung betrifft die Verwendung von synergistischen Desinfektionsmittelzusammensetzungen auf Basis von Aminen und quartären Ammoniumsalzen.

Es sind zahlreiche Desinfektions- und Konservierungsmittelzusammensetzungen auf Basis von Aminen und/oder quartären Ammoniumsalzen bekannt. Diese weisen jedoch im allgemeinen, insbesondere bei höheren Verdünnungen, eine unbefriedigende Wirksamkeit gegen Pilze wie z. B. *Aspergillus niger* auf.

Aufgabe der vorliegenden Erfindung war daher die Verwendung von Desinfektionsmittelzusammensetzungen auf Basis von Aminen und/oder quartären Ammoniumsalzen, welche auch bei hoher Verdünnung eine gute Wirksamkeit gegen Pilze aufweisen.

Erfindungsgemäss wird diese Aufgabe durch die Verwendung nach Patentanspruch 1 gelöst.

Es wurde überraschend gefunden, dass quartäre Ammoniumsalze der allgemeinen Formel (Ib), gegebenenfalls mit Aminen der allgemeinen Formel (Ia) 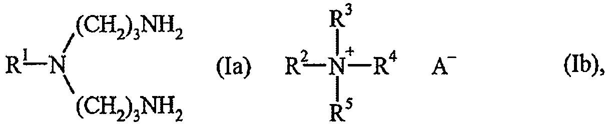
worin R¹ C₆₋₁₈-Alkyl,
R² Benzyl oder C₆₋₁₈-Alkyl,
R³ C₁₋₁₈-Alkyl, oder -[(CH₂)₂-O]*ₙ*R⁵ mit *n* = 1-20
R⁴ und R⁵ unabhängig voneinander C₁₋₄-Alkyl
R⁶ Wasserstoff oder gegebenenfalls substituiertes Phenyl
und A⁻ ein einwertiges Anion oder ein Äquivalent eines mehrwertigen Anions einer anorganischen oder organischen Säure bedeutet;
durch Zusatz von wenigstens einem Alkanolamin der allgemeinen Formel 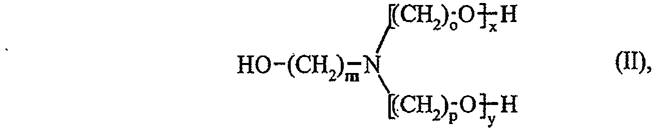
worin m und, soweit vorhanden, o und p unabhängig voneinander den Wert 2 oder 3 und x und y unabhängig voneinander den Wert 0 oder 1 haben, oder einem entsprechenden Salz; im Massenverhältnis (Ib):(II) von 5:1 bis 1:5 eine gute fungizide Wirksamkeit erhalten.

Unter Alkyl sind hier und im folgenden jeweils lineare oder verzweigte Alkylgruppen der angegebenen Kohlenstoffzahlen zu verstehen, vorzugsweise jedoch lineare Alkylgruppen und besonders bevorzugt solche mit gerader Zahl von Kohlenstoffatomen. Insbesondere sind hierunter auch die von natürlichen Rohstoffen abgeleiteten Homologengemische wie beispielsweise "Kokosalkyl" zu verstehen.

Unter substituiertem Phenyl sind insbesondere mit einer oder mehreren C₁₋₈-Allkylgruppen und/oder Chloratomen substituierte Phenylgruppen zu verstehen.

Als Anion A- eignen sich grundsätzlich alle anorganischen oder organischen Anionen, insbesondere Halogenid wie beispielsweise Chlorid oder Bromid oder Anionen niedriger Carbonsäuren wie beispielsweise Acetat, Propionat oder Lactat.

Das Amin bzw. quartäre Ammoniumsalz (Ia/Ib) ist vorzugsweise *N,N*-Bis(3-aminopropyl)-dodecylamin, *N,N*-Bis(3-aminopropyl)octylamin, ein Didecyl-dimethylammoniumsalz, Dioctyl-dimethylammoniumsalz, Octyl-decyl-dimethylammoniumsalz, Dikokosalkyl-dimethylammoniumsalz, Kokosalkyl-dimethyl-poly(oxyethyl)ammoniumsalz, Dikokosalkyl-methyl-po!y(oxyethyl)ammoniumsalz, Decyl-dimethyl-poly(oxyethyl)ammoniumsalz, Didecyl-methyl-poly(oxyethyl)ammoniumsalz, Octyl-dimethyl-poly(oxyethyl)ammoniumsalz, Dioctyl-methyl-poly(oxyethyl)ammoniumsalz, Kokosalkyl-dimethyl-benzylammoniumsalz, Benzyl-dodecyl-dimethylammoniumsalz oder Benzyl-dimethyl-poly(oxyethyl)ammoniumsalz oder ein Gemisch von zweien oder mehreren dieser Verbindungen.

Als Alkanolamine (II) eignen sich grundsätzliche alle Ethanol- und Propanolamine, insbesondere Monoethanolamin, Diethanolamin, Triethanolamin und 3-Amino-1-propanol. Es liegt selbstverständlich auch im Rahmen der Erfindung, Gemische der genannten Verbindungen einzusetzen. Besonders gute Ergebnisse wurden mit den Verbindungen mit primärer Aminogruppe erhalten, nämlich mit Monoethanolamin und 3-Amino-1-propanol.

Die erfindungsgemässen Desinfektionsmittelzusammensetzungen enthalten Wasser als Lösungsmittel, gegebenenfalls in Kombination mit einem organischen Lösungsmittel.

Vorzugsweise enthalten die erfindungsgemässen Desinfektionsmittelzusammensetzungen noch einen oder mehrere Hilfsstoffe aus der Gruppe bestehend aus organischen Lösungsmitteln, Tensiden, Komplexbildnern, Duftstoffen und Farbstoffen.

Ein bevorzugtes Anwendungsgebiet der erfindungsgemässen Desinfektionsmittelzusammensetzungen ist die Flächen- und Instrumentendesinfektion.

Weitere bevorzugte Anwendungsgebiete sind die Wäschedesinfektion und die Händedesinfektion.

Die folgenden Beispiele verdeutlichen die Ausführung der Erfindung, ohne dass darin eine Beschränkung auf die beschriebenen Ausführungsformen zu sehen ist. Alle Mengenangaben sind, soweit nicht anders angegeben, in Massen-%. Als Testkeim wurde jeweils *Aspergillus niger* ATCC 16404 eingesetzt. Die Wirksamkeit wurde, soweit nichts anderes angegeben ist, nach dem in CEN 1275 spezifizierten Verfahren bestimmt.

### Beispiel 1 (nicht erfindungsgemäß)

Es wurde eine Desinfektionsreinigerformulierung (Konzentrat) hergestellt aus:

| | |
|---|---|
| 5,0% | Didecyldimethylammoniumchlorid (50%ige Lösung) |
| 2,0% | *N,N-*Bis(3-aminopropyl)dodecylamin |
| 5,0% | Monoethanolamin |
| 5,0% | Genapol^{®} T250 (Talgfettalkoholpolyglycolether, 25 mol Ethylenoxid) |
| 0,5% | Natriummetasilicat |
| 0,5% | Natriumcarbonat |
| 2,0% | Methylglycindiessigsäure-Trinatriumsalz (Trilon^{®} M; 40%ige Lösung) |
| | Wasser ad 100% |

Die Wirksamkeit wurde mit einer Verdünnung (1 Teil Konzentrat, 99 Teile Wasser) bei 20 °C und 15 min Kontaktzeit bestimmt. Der dekadische Logarithmus der Keimzahlreduktion war 4,1.

### Vergleichsbeispiel 1

Es wurde wie in Beispiel 1 verfahren, jedoch mit dem Unterschied, dass das Monoethanolamin durch die gleiche Menge Wasser ersetzt wurde. Unter den gleichen Testbedingungen war die Formulierung praktisch unwirksam.

### Beispiel 2 (nicht erfindungsgemäß)

Es wurde eine Desinfektionsmittelformulierung (Konzentrat) hergestellt aus:

| | |
|---|---|
| 4,9% | *N,N*-Bis(3-aminopropyl)dodecylamin |
| 4,0% | Monoethanolamin |
| 2,0% | Genapol^{®} T250 (Talgfettalkoholpolyglycolether, 25 mol Ethylenoxid) |
| 5,0% | Hostapur^{®} SAS 30 (C₁₃₋₁₇ sekundäre *n*-Alkansulfonsäure, Natriumsalz) |
| 2,0% | Ethylendiamintetraessigsäure-Tetranatriumsalz (40%ige Lösung) |
| 0,7% | Ethylendiamintetraessigsäure |
| | Wasser ad 100% |

Die Wirksamkeit wurde mit einer Verdünnung (1 Teil Konzentrat, 199 Teile Wasser) bei 20 °C und 15 min Kontaktzeit bestimmt. Der dekadische Logarithmus der Keimzahlreduktion war 4,3.

### Beispiel 3 (nicht erfindungsgemäß)

Es wurde eine Desinfektionsmittelformulierung (Konzentrat) hergestellt aus:

| | |
|---|---|
| 4,2% | *N,N*-Bis(3-aminopropyl)dodecylamin |
| 2,0% | Didecyl-methyl-poly(oxyethyl)ammoniumpropionat (BARDAP 26) |
| 4,0% | Monoethanolamin |
| 2,0% | Genapol^{®} T250 (Talgfettalkoholpolyglycolether, 25 mol Ethylenoxid) |
| 5,0% | Hostapur^{®} SAS 30 (C₁₃₋₁₇ sekundäre *n*-Alkansulfonsäure, Natriumsalz) |
| 2,0% | Ethylendiamintetraessigsäure-Tetranatriumsalz (40%ige Lösung) |
| 0,7% | Ethylendiamintetraessigsäure |
| 4,0% | Butyldiglycol |
| | Wasser ad 100% |

Die Wirksamkeit wurde mit einer Verdünnung (1 Teil Konzentrat, 199 Teile Wasser) bei 20 °C und 15 min Kontaktzeit bestimmt. Der dekadische Logarithmus der Keimzahlreduktion war >4,4.

Zusätzlich wurde die Wirksamkeit noch nach dem in CEN 1650 spezifizierten Verfahren bei einer Kontaktzeit von 15 min, einer Konzentration von 1,0%, einer Wasserhärte von 30 °fH und einer organischen Belastung von 0,3% Albumin bestimmt. Der Logarithmus der Keimzahlreduktion war >4,4.

### Beispiele 4-15

Es wurden wässrige Lösungen aus 0,5% Alkanolamin (II) und 0,25% quartäres Ammoniumsalz (Ib) hergestellt und nach dem in CEN 1275 spezifizierten Verfahren gestestet. Die Ergebnisse sind in Tabelle 1 zusammengefasst.

**Tabelle 1**

| **Beispiel Nr.** | **Amin/Ammoniumsalz** | **Alkanolamin** | **1g Keimreduktion** |
|---|---|---|---|
| 4 | Dimethyl-dioctyl-ammoniumchlorid | Monoethanolamin | 4,3 |
| 5 | dto. | Diethanolamin | 4,0 |
| 6 | dto. | Triethanolamin | 3,6 |
| 7 | dto. | 3-Amino-1-propanol | 4,2 |
| 8 | Didecyl-dimethyl-ammoniumchlorid | Monoethanolamin | 4,0 |
| 9 | dto. | Diethanolamin | 3,8 |
| 10 | dto. | Triethanolamin | 3,1 |
| 11 | dto. | 3-Amino-1-propanol | 4,0 |
| 12 | Di-C₈₋₁₀-alkyldimethyl-ammoniumchlorid (60%) / C₁₂₋₁₆-Alkylbenzyl-dimethylammoniumchlorid (40%); Bardac^{®} 205-M | Monoethanolamin | 3,9 |
| 13 | dto. | Diethanolamin | 3,2 |
| 14 | dto. | Triethanolamin | 2,8 |
| 15 | dto. | 3-Amino-1-propanol | 3,8 |

| | | | |
|---|---|---|---|
| Zum Vergleich wurden alle in Tabelle 1 aufgeführten Verbindungen als Einzelsubstanzen in 0,5%iger Lösung getestet. Keine dieser Verbindungen wies eine ausgeprägte fungizide Wirkung auf (1g Keimreduktion <2). | | | |

## Patentansprüche

1. Verwendung einer Zusammensetzung, bestehend aus
a) einem quartären Ammoniumsalz der allgemeinen Formel 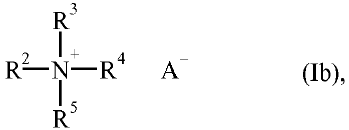 und gegebenenfalls einem Amin der allgemeinen Formel 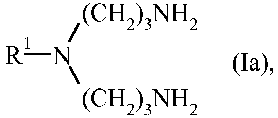
worin R¹ C₆₋₁₈-Alkyl,
R² Benzyl oder C₆₋₁₈-Alkyl,
R³ C₁₋₁₈-Alkyl oder -[(CH₂)₂-O]*ₙ*R⁶ mit n = 1-20,
R⁴ und R⁵ unabhängig voneinander C₁₋₄-Alkyl,
R⁶ Wasserstoff oder gegebenenfalls substituiertes Phenyl und A⁻ ein einwertiges Anion oder ein Äquivalent eines mehrwertigen Anions einer anorganischen oder organischen Säure bedeutet;
b) wenigstens einem Alkanolamin der allgemeinen Formel 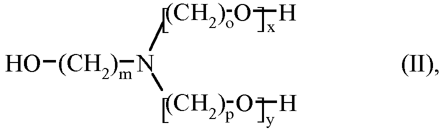 worin m und, soweit vorhanden, o und p unabhängig voneinander den Wert 2 oder 3 und x und y unabhängig voneinander den Wert 0 oder 1 haben, oder einem entsprechenden Salz;
im Massenverhältnis (Ib):(II) von 5:1 bis 1:5;
c) Wasser als Lösungsmittel;
sowie gegebenenfalls einem oder mehreren Hilfsstoffen aus der Gruppe bestehend aus organischen Lösungsmitteln, Tensiden, Komplexbildnern, Duftstoffen und Farbstoffen; als nicht-therapeutisches Desinfektionsmittel gegen Pilze.

2. Verwendung nach Anspruch 1, **dadurch gekennzeichnet, dass** das Amin bzw. quartäre Ammoniumsalz ausgewählt ist aus der Gruppe bestehend aus *N,N*-Bis(3-aminopropyl)-dodecylamin, *N,N*-Bis(3-aminopropyl)octylamin, Didecyl-dimethylammoniumsalzen, Dioctyl-dimethylammoniumsalzen, Octyl-decyl-dimethylammoniumsalzen, Kokosalkyldimethyl-benzylammoniumsalzen und Benzyl-dimethyl-oxethylammoniumsalzen sowie Gemischen dieser Verbindungen.

3. Verwendung nach Anspruch 1 oder 2, **dadurch gekennzeichnet, dass** das Alkanolamin (II) ausgewählt ist aus der Gruppe bestehend aus Monoethanolamin, Diethanolamin, Triethanolamin und 3-Amino-1-propanol.

4. Verwendung gemäss Ansprüchen 1 bis 3 zur Flächen- und Instrumentendesinfektion.

5. Verwendung gemäss Ansprüchen 1 bis 3 zur Wäschedesinfektion.

6. Verwendung gemäss Ansprüchen 1 bis 3 zur Händedesinfektion.

## Claims

1. Use of a composition consisting of
a) a quaternary ammonium salt of the general formula 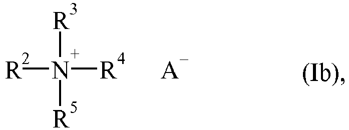 and, optionally, an amine of the general formula 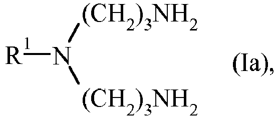
wherein R¹ is C₆₋₁₈ alkyl,
R² is benzyl or C₆₋₁₈ alkyl,
R³ is C₁₋₁₈ alkyl or -[(CH₂)₂-O]*ₙ*R⁶ where n = 1-20,
R⁴ and R⁵ independently of one another are C₁₋₄ alkyl,
R⁶ is hydrogen, or optionally substituted phenyl,
and A⁻ is a monovalent anion or one equivalent of a polyvalent anion of an inorganic or organic acid;
b) at least one alkanolamine of the general formula 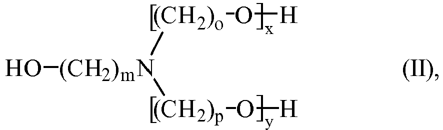 wherein m and, if present, o and p independently of one another have the value 2 or 3 and x and y independently of one another have the value 0 or 1, or a corresponding salt;
in the mass ratio (Ib):(II) of 5:1 to 1:5;
c) water as solvent;
and, optionally, one or more adjuvants selected from the group consisting of organic solvents, surfactants, complexing agents, fragrances and colorants;
as a non-therapeutic disinfectant against fungi.

2. Use according to claim 1, **characterized in that** the amine and the quaternary ammonium salt are selected from the group consisting of *N,N*-bis(3-aminopropyl)dodecylamine, *N,N*-bis(3-aminopropyl)octylamine, didecyl-dimethylammonium salts, dioctyl-dimethylammonium salts, octyl-decyl-dimethylammonium salts, cocoalkyl-dimethyl-benzyl-ammonium salts, benzyl-dimethyl-oxethylammonium salts, and mixtures of these compounds.

3. Use according to claim 1 or 2, **characterized in that** the alkanolamine (II) is selected from the group consisting of monoethanolamine, diethanolamine, triethanolamine and 3-amino-1-propanol.

4. Use according to claims 1 to 3 for surface disinfection and instrument disinfection.

5. Use according to claims 1 to 3 for laundry disinfection.

6. Use according to claims 1 to 3 for hand disinfection.

## Revendications

1. Utilisation d'une composition, constituée par :
a) un sel d'ammonium quaternaire de formule générale 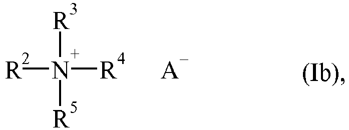 et éventuellement une amine de formule générale 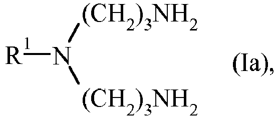
dans lesquelles R¹ signifie alkyle en C₆₋₁₈,
R² signifie benzyle ou alkyle en C₆₋₁₈,
R³ signifie alkyle en C₁₋₁₈ ou -[(CH₂)₂-O]*ₙ*R⁶ avec n = 1 à 20,
R⁴ et R⁵ signifient indépendamment l'un de l'autre alkyle en C₁₋₄,
R⁶ signifie hydrogène ou phényle éventuellement substitué,
et A⁻ signifie un anion monovalent ou un équivalent d'un anion polyvalent d'un acide inorganique ou organique ;
b) au moins un alcanolamine de formule générale 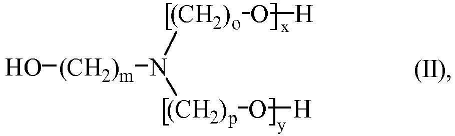 dans laquelle m et, le cas échéant, o et p, ont indépendamment les uns des autres la valeur 2 ou 3, et x et y ont indépendamment l'un de l'autre la valeur 0 ou 1, ou un sel correspondant ;
en un rapport en masse (Ib):(II) de 5:1 à 1:5 ;
c) de l'eau en tant que solvant ;
ainsi qu'éventuellement un ou plusieurs adjuvants du groupe constitué par les solvants organiques, les tensioactifs, les agents complexants, les parfums et les colorants ;
en tant qu'agent désinfectant non thérapeutique contre les champignons.

2. Utilisation selon la revendication 1, **caractérisée en ce que** l'amine ou le sel d'ammonium quaternaire est choisi dans le groupe constitué par la *N,N*-bis(3-aminopropyl)-dodécylamine, la N,N-bis(3-aminopropyl)-octylamine, les sels de didécyl-diméthylammonium, les sels de dioctyl-diméthylammonium, les sels d'octyl-décyl-diméthylammonium, les sels de alkyl-diméthyl-benzylammonium de coco et les sels de benzyl-diméthyl-oxéthylammonium, ainsi que les mélanges de ces composés.

3. Utilisation selon la revendication 1 ou 2, **caractérisée en ce que** l'alcanolamine (II) est choisie dans le groupe constitué par la monoéthanolamine, la diéthanolamine, la triéthanolamine et le 3-amino-1-propanol.

4. Utilisation selon les revendications 1 à 3 pour la désinfection de surfaces et d'instruments.

5. Utilisation selon les revendications 1 à 3 pour la désinfection de linge.

6. Utilisation selon les revendications 1 à 3 pour la désinfection des mains.
